# EUROPÄISCHE PATENTSCHRIFT

(11) **EP 0 531 250 B1**
(45) Veröffentlichungstag und Bekanntmachung des Hinweises auf die Patenterteilung: **08.11.1995**
(21) Anmeldenummer: 92810452.0
(22) Anmeldetag: 11.06.1992
(51) Int. Cl.: A61F 2/38

(54) **Kniegelenkprothese**
Knee joint prosthesis
Prothèse d'articulation du genou

(30) Priorität: 28.08.1991 CH 2523/91
(43) Veröffentlichungstag der Anmeldung: 10.03.1993
(73) Patentinhaber: SULZER Medizinaltechnik AG, 8404 Winterthur (CH); PROTEK AG, 3110 Münsingen-Bern (CH)
(72) Erfinder: Aubriot, Jacques-Hubert, F-14000 Caen (FR); Roland, Willi, CH-8413 Neftenbach (CH)
(74) Vertreter: Hammer, Bruno, Dr.

(56) Entgegenhaltungen:
- GB-A- 2 120 943
- US-A- 4 257 129
- US-A- 4 808 185

## Beschreibung

Kniegelenkprothese mit einem Tibiaplateau aus Polyethylen, das in einem metallenen Tibiaschaft mit Teller eingebettet ist und das zwei Führungsflächen für die Kondylen eines Femurteiles aufweist, wobei sich der Teller in Richtung der Schaftachse auf einer vorbereiteten Stirnfläche der Tibia abstützt, und wobei die Tellerunterseite eben ausgeführt ist und auf einer als ebene Stirnfläche ausgeführten Resektionsfläche abstützbar ist. Eine solche Kniegelenkprothese ist aus der US-A-4257129 bekannt.

Die US-A-4,568,348 zeigt eine Kniegelenkprothese mit konischem Teller und mit konischem Unterteil des Tibiaplateau's. Ein Problem derartiger Kniegelenkprothesen besteht darin, dass relativ viel gesundes Knochenmaterial entfernt werden muss, um die Prothese einzusetzen. Im weiteren sind konische und nicht rotationssymmetrische Verankerungsflächen schwierig so herzustellen, dass sie neben der Formgenauigkeit auch noch in der richtigen Höhe und Winkellage zur Längsachse der Tibia stehen. Hier schafft die Erfindung Abhilfe.

Aufgabe der Erfindung ist es, eine Prothese mit wenig Verlust an tragendem Knochenmaterial zu schaffen, deren Gegenflächen im Knochenmaterial einfach herzustellen sind. Diese Aufgabe wird durch die Erfindung dadurch gelöst, dass auf der Tellerinnenseite im Bereich der Führungsflächen Taschen angebracht sind, die in sagittalen Ebenen der Wölbung der Führungsflächen folgen, so daß über einen größeren Winkelbereich eine annähernd konstante Wandstärke für das Tibiaplateau entsteht, und die an ihrem tiefsten Punkt eine Wandstärke von maximal 1,5 mm zur Resektionsfläche aufweisen, während die Mindestwandstärke des in den Taschen anliegenden Tibiaplateau's im Bereich der Taschen 6,5 mm beträgt.

Die Vorteile der Erfindung sind darin zu sehen, dass zwischen den Führungsflächen zu den Kondylen und der Resektionsebene ein minimaler Abstand eingehalten wird, aufgrund dessen genügend Knochenmaterial für eine optimale Abstützung und für eine spätere Revisionsprothese zu Verfügung steht.

Im folgenden wird die Erfindung anhand von einem Ausführungsbeispiel beschrieben. Es zeigen:
- Figur 1:: Die Ansicht eines metallenen Tibiaschaftes mit den Aufnahmeflächen für ein Tibiaplateau und
- Figur 2:: Die Seitenansicht eines Vertikalschnittes durch eine in der Tibia eingesetzte Kniegelenkprothese mit Tibiaplateau, mit Tibiaschaft und mit Teller.

In den Figuren ist eine Kniegelenkprothese mit zwei Führungsflächen 4 für die Kondylen eines Femurteils gezeigt. Die Prothese besteht aus einem Tibiaplateau 1 aus Polyethylen, das in einem metallenen Tibiaschaft 2 mit Teller 3 eingebettet ist. Die Tellerunterseite ist eben und stützt sich auf einer als ebene Stirnfläche 6 ausgeführte Resektionsfläche der Tibia 7 ab. Auf der Tellerinnenseite sind im Bereich der Führungsflächen 4 Taschen 8, 9 angebracht, die in sagittalen Ebenen der Wölbung der Führungsflächen 4 folgen und die an ihrem tiefsten Punkt eine Wandstärke 10 von maximal 1,5 mm zur Resektionsfläche 6 aufweisen, während die Mindestwandstärke des in den Taschen 8, 9 anliegenden Tibiaplateau's 1, 6,5 mm beträgt.

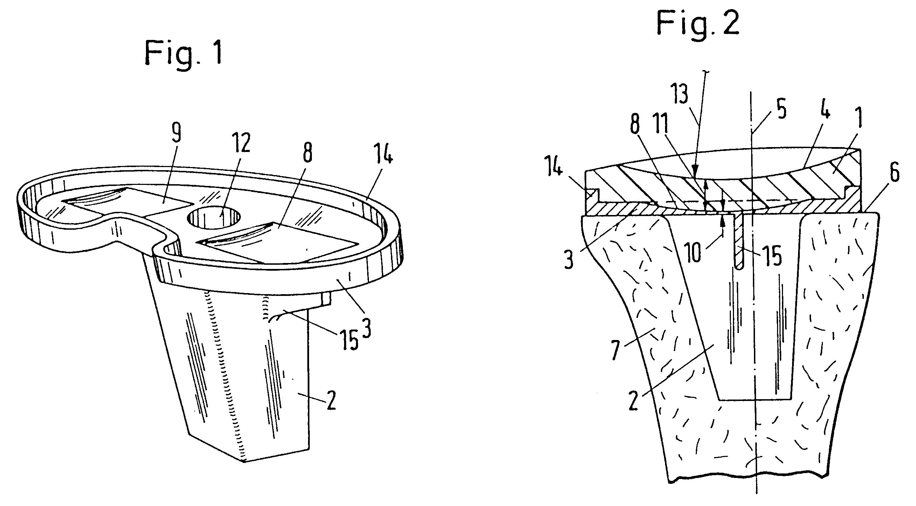

In Figur 1 ist an einen Schaft 2 ein ebener Teller 3 angeformt, der sich auf einer ebenen Resektionsfläche 6 abstützen kann. Der konisch zulaufende Schaft 2 hat Rechteckquerschnitt um auch Torsionskräfte formschlüssig zu übertragen. Verstärkungsrippen 15, die parallel zur Schaftachse 5 verlaufen können ebenfalls Torsionskräfte in der Schaftachse übertragen und verstärken zusätzlich den Uebergang Schaft-Teller. Der Teller 3 ist von einem Rand 14 umgeben, in dem das Tibiaplateau 1 formschlüssig eingelegt wird. Auf der Tellerinnenseite sind Taschen 8, 9 angebracht, die unterhalb von den führungsflächen 4 für die Kondylen liegen. Die Wandstärke 10 zwischen dem tiefsten Punkt der Taschen 8, 9 und der Stirnfläche 6 beträgt maximal 1,5 mm, um die Resektionsfläche möglichst hoch an der Tibia ansetzen zu können. Andererseits ist gemäss Fig. 2 im Belastungsbereich des Tibiaplateau's eine Mindestwandstärke 11 von 6,5 mm notwendig, um für den Werkstoff Polyethylen formbeständige führungsflächen zu erhalten. In sagittalen Ebenen folgen die Taschen 8, 9 der Wölbung der führungsflächen 4, so dass über einen grösseren Winkelbereich eine annähernd konstante Wandstärke für das Tibiaplateau 1 entsteht. Auf diese Weise werden auch Gelenkkräfte, die nicht genau in der Schaftachse liegen in diesem Winkelbereich als Normalkräfte zwischen Tibiaplateau 1 und Teller 3 übertragen. Dies setzt voraus, dass das Tibiaplateau 1 in den Taschen 8, 9 anliegt. Kraftverschiebungen dieser Art ergeben sich, wenn die Kondylen mit einem kleineren Radius als der Radius 13 der Führungsfläche 4 an dieser anliegen. Beim Einsetzen des Tibiaschaftes 2 wird dieser beispielsweise über eine Befestigungsbohrung 12 mit einem nicht gezeigten Verankerungsschaft zur Primärverankerung verbunden.

## Patentansprüche

1. Kniegelenkprothese mit einem Tibiaplateau (1) aus Polyethylen, das in einem metallenen Tibiaschaft (2) mit Teller (3) eingebettet ist und das zwei Führungsflächen (4) für die Kondylen eines Femurteiles aufweist, wobei sich der Teller in Richtung der Schaftachse (5) auf einer vorbereiteten Stirnfläche (6) der Tibia (7) abstützt, und wobei die Tellerunterseite eben ausgeführt ist und auf einer als ebene Stirnfläche (6) ausgeführten Resektionsfläche abstützbar ist, **dadurch gekennzeichnet,** dass auf der Tellerinnenseite im Bereich der Führungsflächen (4) Taschen (8, 9) angebracht sind, die in sagittalen Ebenen der Wölbung der Führungsflächen (4) folgen, so daß über einen größeren Winkelbereich eine annähernd konstante Wandstärke für das Tibiaplateau (1) entsteht, und die an ihrem tiefsten Punkt eine Wandstarke (10) von maximal 1,5 mm zur Resektionsfläche (6) aufweisen, während die Mindestwandstärke (11) des in den Taschen (8, 9) anliegenden Tibiaplateau's (1) 6,5 mm beträgt.

2. Kniegelenkprothese nach Anspruch 1, dadurch gekennzeichnet, dass die Unterseite des Tellers (3) in der Nähe des tiefsten Punktes der Taschen (8, 9) eine Verstärkungsrippe (15) von lateral nach medial aufweist.

## Claims

1. A kneejoint prosthesis having a tibia plateau (1) of polyethylene which is embedded by a plate (3) in a metal tibia shank (2), and exhibits two guidefaces (4) for the condyles of one part of the femur, whilst the plate bears in the direction of the axis (5) of the shank against a prepared endface (6) of the tibia (7), the underside of the plate being made plane and able to bear against a resection area made as a plane endface (6)
characterized in that on the inside of the plate in the region of the guidefaces (4) pockets (8, 9) are placed, which follow in sagittal planes the curvature of the guidefaces (4) so that over a greater range of angle an approximately constant thickness of wall arises for the tibia plateau (1), the said pockets exhibiting at their lowest point a wall thickness (10) of a maximum of 1.5 mm to the resection area (6), whilst the minimum wall thickness (11) of the tibia plateau (1) resting in the pockets (8, 9) amounts to 6.5 mm.

2. A kneejoint prosthesis as in Claim 1, characterized in that the underside of the plate (3) exhibits near the lowest point of each pocket (8, 9) a strengthening rib (15) from lateral to medial.

## Revendications

1. Prothèse d'articulation du genou avec un plateau de tibia (1) en polyéthylène, qui est noyé dans une tige de tibia (2) métallique avec platine (3) et qui présente deux surfaces de guidage (4) pour les condyles d'une partie de fémur, la platine prenant appui en direction de l'axe (5) de la tige sur une surface frontale (6) préparée du tibia (7) et la face inférieure de la platine étant plane et étant apte à s'appuyer sur une surface de résection réalisée sous la forme d'une surface frontale (6) plane, caractérisée en ce que sur la face intérieure de la platine, dans la région des surfaces de guidage (4), sont appliquées des poches (8, 9) qui, dans des plans sagittaux, suivent la courbure des surfaces de guidage (4), de sorte que sur une grande plage angulaire il se forme une épaisseur de paroi à peu près constante pour le plateau de tibia (1), et qui présentent, à leur point le plus bas, une épaisseur de paroi (10) de 1,5 mm au maximum par rapport à la surface de résection (6), tandis que l'épaisseur minimale de paroi (11) du plateau de tibia (1), s'appliquant dans les poches (8, 9), est de 6,5 mm.

2. Prothèse d'articulation du genou selon la revendication 1, caractérisée en ce que la face inférieure de la platine (3), au voisinage du point le plus bas des poches (8, 9), présente une nervure de renfort (15), du côté latéral au côté médial.
